# EUROPEAN PATENT APPLICATION

(11) **EP 3 950 936 A1**
(43) Date of publication of application: **09.02.2022**
(21) Application number: 20778384.6
(22) Date of filing: 23.03.2020
(51) Int. Cl.: C12N 5/0783, A61K 8/98, A61K 35/17, A61P 17/00, A61P 37/02, A61Q 19/00

(54) **ADDITIVE COMPOSITION FOR NK CELL CULTURE MEDIUM, CULTURE METHOD FOR NK CELL BY USING SAME ADDITIVE COMPOSITION, AND COSMETICS COMPOSITION OBTAINED THEREBY FOR IMPROVING SKIN PROBLEMS**

(30) Priority: 27.03.2019 KR 20190035275
(71) Applicant: Shin, Ji Seop, Seoul 04355 (KR); Shin, Woo Seop, Gyeonggi-do 13527 (KR)
(72) Inventor: JANG, Min Ji, Seoul 04355 (KR); SHIN, Dong Hyuk, Seongnam-si, Gyeonggi-do 13527 (KR); SHIN, Ji Seop, Seoul 04355 (KR); SHIN, Woo Seop, Seongnam-si, Gyeonggi-do 13527 (KR)
(74) Representative: Beck & Rössig European Patent Attorneys
(86) International application number: PCT/KR2020/003943
(87) International publication number: WO 2020/197216

(57) **Abstract**

Disclosed is a method of culturing natural killer (NK) cells used in immunotherapy. Specifically, an additive composition for an NK cell culture medium is disclosed. The additive composition inhibits the activity of CD4+ T cells, thereby effectively helping NK cell proliferation and activation. In addition, an NK cell culture method using the additive composition is disclosed. In addition, a cosmetic composition for alleviating skin troubles is disclosed, and the cosmetic composition is prepared using the NM cell culture method.

## Description

### Technical Field

The present invention relates to a method for culturing natural killer (NK) cells used in immunotherapy. More specifically, the present invention relates to an additive composition for an NK cell culture medium to effectively amplify and activate NK cells by inhibiting regulatory T cells (Tregs), a method for culturing NK cells using the additive composition, and a cosmetic composition prepared by the method for alleviating skin troubles.

### Background Art

NK cells are known to play an important role in the initial body defense mechanism and tumor immunity in the human body. That is, NK cells can kill specific autologous cells, allogeneic cells, and even heterogeneous cancer cells without an immunity acquisition process caused by the expression of a major histocompatibility complex (MHC). Especially, NK cells can better kill target cells that never or rarely expresses Class1 MHC. Therefore, NK cells can effectively kill most cancer cells in which MHC is not expressed and also can kill some virus-infected cells and bacteria such as salmonella typhi. However, NK cells, which have such an excellent effect of killing cancer cells, occupy only 5 to 15% of peripheral blood lymphocytes even in normal human bodies. The percentage of NK cells is decreased to below 1% in the case of patients with severe cancer stages. Therefore, there is a limit in that NK cells effectively attacks cancer cells without an amplification process performed in immunotherapy.

Immunotherapy involves extracting the most important immune cells for cancer treatment, such as natural killer (NK) cells, dendritic cells (DC), B cells, and T cells, from the patient's blood, growing the extracted cells strong immune cells that can effectively active on cancer cells using various types of stimulants, and injecting them back into the patient body. Since the patient's own blood is used, the immunotherapy causes fewer side effects than conventional chemotherapy and has the advantage of an easy and simple administration method, it has been actively studied and researched in recent years.

For the culture of NK cells, the help of T cells, especially helper T cells, is required. Helper T cells (also called T helper cells or Tₕ cells) refer to cells that promote humoral and cellular immunity by regulating the differentiation and activation of white blood cells. Helper T cells are also called CD4+ T cells because they express a protein called CD4 on their surface. CD4+ T cells are classified into Th1, Th2, Th17, and Treg cells according to their functions. Th1 cells secrete interferon-gamma (IFN-γ) and tumor necrosis factor beta (TNF-β) to induce the fusion of endosomes and lysosomes in macrophages to form endolysosomes. Th2 cells secrete several types of interleukin (IL) to differentiate B cells into plasma cells. Th17 cells secrete interleukin-17 (IL-17) to attract neutrophils. Treg cells, also called regulatory T cells, do not promote an immune response, but rather suppress it, thereby maintaining immune homeostasis and blocking autoimmune responses. Regulatory T cells (Tregs) are components of the immune system and suppress the immune response of other cells. This is an important 'self-check' action designed to prevent overreaction in the immune system. Regulatory T cells are involved in stopping the immune response after successfully killing invader microorganisms, and are also involved in preventing autoimmune diseases. The immune system must be able to distinguish between self and non-self. When the immune system fails to distinguish between self and non-self, the immune system destroys cells and tissues in the body, resulting in autoimmune diseases. Regulatory T cells actively suppress immune response and prevent pathological autoimmunity such as autoimmune diseases. The molecular-level working principle of regulatory T cells has not yet been clearly elucidated. That is, there has not been any report on how regulatory T cells perform inhibitory and regulatory actions, but it has been reported that the immunosuppressive cytokine TGF-beta and the interleukin 10 (IL-10) are associated with the functions of regulatory T cells.

On the other hand, when various types of antibodies or cytokines are used to activate immune cells, regulatory T cells (also called Treg cells or Tregs) are also activated. However, it takes a longer time for the Tregs to be activated than for other cells. Therefore, the number of cells increases fast in the early stage of cell culture, but the number of cells does not increase as much as expected due to the activated Tregs when the cells are cultured for a period of about 8 to 9 days or more. This phenomenon is often found in cancer patients with weakened immunity. The phenomenon is conspicuously increasingly found in patients who have a large number of Treg cells and in which Tregs are activated, because of the effect of Tregs.

Therefore, it is required to develop a technology for the mass culture of NK cells, the technology being capable of promoting the proliferation of immune cells in large quantities while minimizing the effect of Tregs.

### Disclosure

### Technical Problem

The inventors of the present application have made intensive research and efforts to solve the above problems, and thus have developed a technique capable of inhibiting the activity of regulatory T cells (Tregs) during the culture of immune cells. As a result, the inventions disclosed in the present invention have been made.

Accordingly, an objective of the present invention is to provide an additive composition for a culture medium for NK cells, the composition capable of suppressing the activity of CD4+T cells, especially regulatory T cells (Tregs), in a specific period during the culture of NK cells, thereby promoting the proliferation of the NK cells. In addition, it is intended to provide an NK cell culture method using the same composition and an immune cell therapeutic agent.

Another objective of the present invention is to provide a cosmetic composition including a serum-free immune cell culture medium with high concentrations of IFN-γ and IL-10 as an active ingredient, thereby effectively treating inflammation and alleviating skin troubles.

A further objective of the present invention is to provide a pharmaceutical composition containing a serum-free immune cell culture medium with high concentrations of IFN-γ and IL-10 as an active ingredient, the composition having the effect of removing inflammation, thereby being effective in treating injuries such as wounds and burns and in treating skin troubles.

The objectives of the present invention are not limited to the above-mentioned objectives, and other objectives that can be recognized by the ordinarily skilled in the art from the description in the following detailed description section should be regarded as the objectives of the present invention although not explicitly mentioned in this section.

### Technical Solution

In order to achieve the objectives of the present invention described above, one aspect of the present invention is to provide an additive composition for an NK cell culture medium, the additive composition including one or more Treg suppressing substances as an active ingredient.

In a preferred embodiment, the active ingredient may be a steroid that enables the proliferation of NK cells.

In a preferred embodiment, the steroids may be glucocorticoids.

In a preferred embodiment, the glucocorticoid may include at least one selected from the group consisting of cortisol, cortisone, prednisolone, prednisone, methylprednisolone, dexamethasone, betamethasone, triamcinolone, fludrocortisone acetate, and deoxycorticosterone acetate.

In a preferred embodiment, the additive composition may be treated after T cells in the NK cell culture medium are stimulated by an NK cell culturing process.

In a preferred embodiment, the steroid may be contained in a concentration of 0.2 µg/ml or more.

Another aspect of the present invention is to provide a method of culturing NK cells, the method comprising: an NK cell stimulation step of treating a culture medium in which peripheral blood mononuclear cells (PBMCs) isolated from blood are cultured with an NK cell stimulation substance; a T cell stimulation step of treating the culture medium with a T cell stimulation substance after the NK cells are stimulated in the NK cell stimulation step; and an additive composition treatment step of treating the culture medium with an additive composition for a NK cell culture medium, the additive composition including one or more T reg activity suppressing substances, after the T cells are stimulated in the T cell stimulation step.

In a preferred embodiment, the NK cell stimulation substance may be at least one selected from the group consisting of an anti-CD16 antibody, an anti-CD56 antibody, and interleukins IL-2, IL-12, and IL-18, and the T cell stimulation substance may be at least one selected from the group consisting of a CD3 antibody, an anti-CD4 antibody, and an anti-CD28 antibody.

In a preferred embodiment, the Treg activity suppressing substances may be steroids and improve the proliferation of the NK cells by inhibiting the immunosuppressive activity of regulatory T cells (Tregs).

In a preferred embodiment, the T cell stimulation step may be performed on the second day of culture, and the additive composition treatment step may be performed on the third day of culture.

In a preferred embodiment, the additive composition treatment step may be performed at least one time on or after the fifth day of culture.

In a preferred embodiment, the additive composition contains the steroid at a concentration of 0.2 ug/mL or more, and the steroid is dissolved in a carrier or cytokine 1-1 solution, wherein the cytokine 1-1 solution is obtained by dissolving IL-12 and IL-18 in a basic solution such that the IL-12 and the IL-18 are contained at a concentration of 0.5-5 ng/mL and a concentration of 2 of 50 ng/mL, respectively in the basic solution.

In a preferred embodiment, the method may further include a step of culturing NK cells for 11 to 16 days after the additive composition treatment step is performed and harvesting the cultured NK cells.

A further aspect of the present invention is to provide an immune cell therapeutic agent comprising the NK cells obtained by one of the above-described NK cell culture methods as an active ingredient.

A further aspect of the present invention is to provide a NK cell reactivation method comprising steps of: preparing exhausted NK cells that are isolated after being cultured for at least 20 days by any one of the above-described NK cell culture methods, NK cells that are harvested, frozen and then thawed; and culturing the prepared exhausted NK cells in a cytokine 1-1 solution.

A yet further aspect of the present invention is to provide a method of preparing an NC cell culture medium composition, the method comprising steps of: isolating the NK cells after culturing the NK cells for 11 days by any one of the NK cell culture methods described above; culturing the isolated NK cells in a cytokine 1-1 solution; and harvesting the NK cells and obtaining the remaining culture medium composition.

A yet further aspect of the present invention is to provide a cosmetic composition for alleviating skin troubles, the cosmetic composition comprising the NK cell culture medium composition obtained by the method of preparing the NK cell culture medium composition as an active ingredient.

In addition, the present invention provides a pharmaceutical composition for the treatment of skin diseases comprising the NK cell medium composition obtained by the above-described method for preparing the NK cell medium composition as an active ingredient.

### Advantageous Effects

The additive composition for NK cell culture medium, according to the present invention, can increase the proliferation rate of NK cells by inhibiting the immunosuppressive activity of CD4+ T cells, specifically regulatory T cells (Tregs), at a specific time during NK cell culture.

In addition, the cosmetic composition and pharmaceutical composition of the present invention contain, as an active ingredient, a serum-free immune cell culture medium with high concentrations of IFN-γ and IL-10, and thus effectively treat inflammation. Therefore, the cosmetic composition and the pharmaceutical composition has excellent effects on wound healing and skin disease treatment.

These technical effects of the present invention are not limited only to the effects mentioned above. Even though not explicitly mentioned above, the effects that can be recognized by those who are ordinarily skilled in the art from the following detailed description of specific embodiments are regarded as the effects provided by the present invention.

### Description of Drawings

FIG. 1 is a graph showing changes in the number of cells over time when cells are cultured by an NK cell culture method according to an embodiment of the present invention;
FIG. 2A is a graph showing the FACS data of culture media used in exemplary NK cell culture methods of the present invention, and FIG. 2B is a graph showing the FACS data of a culture medium prepared according to a comparative example;
FIGS. 3A and 3B are graphs showing the FACS data of culture media that are treated with an additive composition for an NK cell culture medium on the third day, fifth day, and eighth day or culture media that are not treated with the additive composition, upon culturing cells having an NK cell ratio of about 50%, in which the concentration of a steroid in the additive composition is increased two times;
FIG. 4A is a graph showing the FACS data of a culture medium treated with an additive composition for an NK cell culture medium when NK cells are cultured with only an cytokine and an anti-CD3 antibody, and FIG. 4B is a graph showing the FACS data of an untreated culture medium; and
FIG. 5A is a graph showing the FACS data of a culture medium treated with an additive composition for an NK cell culture medium during NK cell culture by a method of immobilizing an anti-CD3 antibody, and FIG. 5B is a graph showing the FACS data of an untreated culture medium.

### Best Mode

The terminology used herein is for the purpose of describing particular embodiments only and is not intended to limit the claimed invention. As used herein, the singular forms "a", "an", and "the" are intended to include the plural forms as well, unless the context clearly indicates otherwise. It will be further understood that the terms "comprises" and/or "comprising," or "includes" and/or "including," or "has" and/or "having" when used in this specification, specify the presence of stated features, regions, integers, steps, operations, elements and/or components, but do not preclude the presence or addition of one or more other features, regions, integers, steps, operations, elements, components and/or groups thereof.

Terms such as first and second are used only for the purpose of distinguishing one component from other components. For example, the first component may be referred to as a second component without departing from the scope of the present invention, and similarly, the second component may be referred to as a first component.

In addition, unless otherwise defined, all terms including technical and scientific terms used herein have the same meaning as commonly understood by those who are ordinarily skilled in the art to which this invention belongs. It will be further understood that terms, such as those defined in commonly used dictionaries, should be interpreted as having a meaning that is consistent with their meaning in the context of the relevant art and the present invention, and will not be interpreted in an idealized or overly formal sense unless expressly so defined herein.

In the present invention, the term "immune cells" is used to include only NK cells and T cells. The term "NK cell" is used to refer to both NK cells and NKT cells, but in some cases, the term "NK cell" may mean only NK cells.

When describing a temporal relationship, that is, a temporal order is expressed with "after", "following", "next", "before", etc., there may be a case where events are not temporally continuous unless expressed with "immediately" or "directly".

Hereinafter, the technical configuration of the present invention will be described in detail with reference to the accompanying drawings and preferred embodiments.

However, the present invention is not limited to embodiments described herein and may be embodied in other forms. Like reference numerals are used to refer to like elements throughout the description of the present application.

The technical feature of the present invention relates to a new use of a drug for suppressing the activity of regulatory T cells (Tregs). Particularly, the present invention features an additive composition for an NK cell culture medium, in which the additive composition includes one or more regulatory T cell (Treg) activity inhibitors as an active ingredient, thereby inhibiting the immunosuppressive activity of CD4+ T cells, particularly regulatory T cells (Tregs), at a specific time during the culture of NK cells, to increase the proliferation of NK cells. In addition, the present invention also features an NK cell culture method using the same additive composition, an immune cell therapeutic agent obtained by the method, and a culture medium composition.

That is, when culturing NK cells, NK cells need to be stimulated not only at the beginning of culture thereof but also during the culture. However, for T cells, initial stimulation of is sufficient. Therefore, when a method is found in which NK cells are first stimulated with an NK cell stimulation substance, T cells are next stimulated with a T cell stimulation substance so as to be activated, the activated T cells and the NK cell stimulation substance then further stimulate the NK cells together, and the immunosuppressive activity of CD4+ T cells, especially regulatory T cells (Tregs) among the activated T cells, is inhibited immediately thereafter, whereby it is possible to activate or proliferate a larger number of NK cells.

Based on this fact, the present invention provides a new use of a regulatory T cell (Treg) activity inhibitor. According to the present invention, a regulatory T cell (Treg) activity inhibitor that is known to inhibit the activity of CD4+ T cells, especially regulatory T cells (Treg), is used to culture NK cells.

More specifically, steroids, widely known as drugs for inhibiting regulatory T cell (Treg) activity, inhibit the activity of CD4+ T cells such as Th1, Th2, Th17, and Treg, thereby treating inflammation and eliminating pain. However, it is also known that the steroids have serious side effects such as reducing immunity when used for a long time. However, in NK cell culture, when the activation of Treg cells is suppressed by steroids after immune cell are activated, NK cell proliferation and activation are not suppressed. That is, it is reported that even though immune cells are overcrowded during the culture of NK cells, the immune cells do not interfere with NK cell activation and proliferation.

Therefore, the additive composition for an NK cell culture medium, according to the present invention, comprises one or more T reg activity suppressing substances as an active ingredient.

Here, as the active ingredient, any known agent can be used if it can inhibit the activity of regulatory T cells (Treg). In one embodiment, steroids can be used as the active ingredient. Steroids inhibit the immunosuppressive activity of CD4+ T cells, especially regulatory T cells (Tregs), thereby affecting the proliferation of NK cells. It is known through experiments that steroids suppress the growth of T cells to some extent by removing the suppressive activity of regulatory T cells in the immune system.

As the steroid contained in the additive composition for an NK cell culture medium, any known steroid that can act as a steroid can be used, but preferably glucocorticoids may be used in the present invention. According to one embodiment, at least one selected from the group consisting of cortisol, cortisone, prednisolone, prednisone, methylprednisolone, dexamethasone, betamethasone, triamcinolone, fludrocortisone acetate, and deoxycorticosterone acetate may be used.

In the additive composition for an NK cell culture medium of the present invention, the Treg activity suppressing substance may be contained at a concentration of 0.1 µg/ml or more. Through experimental examples described later, it was confirmed that the steroid used as an example of the Treg activity suppressing substance had no effect on the proliferation of cellular immune cells such as activated NK cells and Tc cells. It was also confirmed that a ratio of NK cells increased with time. Specifically, when the concentration of the steroid in the additive composed was increased, since the proliferation rate of T cells was suppressed to some extent, the ratio of NK cells was increased in proportion to the concentration of the steroid. When the steroid used as the Treg activity suppressing substance was included at a concentration of less than 0.1 µg/ml, the property of inhibiting the immunosuppressive activity of CD4+ T cells, especially regulatory T cells (Tregs), was not sufficiently exhibited. Through the experimental examples described later, in the case where the steroid was included at a high concentration, if the concentration exceeded 9 µg/ml, it was expected that the effect on the NK cell culture would not change significantly. Therefore, the Treg activity suppressing substance contained in the additive composition for an NK cell culture medium of the present invention is expected to provide the desired effect as long as the concentration thereof is 0.1 µg/ml or higher.

In addition, as is known, when NK cells are cultured, the help of stimulated T cells is needed to sufficiently activate NK cells after the NK cells are stimulated in an early culture stage. Therefore, the additive composition of the present invention may be used after T cells in an NK cell culture medium are stimulated, in the process of culturing NK cells. In this way, when culturing NK cells, in the case where the additive composition for an NK cell culture medium of the present invention is used at an appropriate time after the stimulation of T cells in the NK cell culture medium, it is possible to effectively increase the proliferation rate of Tc and NK immune cells, especially, the proliferation rate of NK cells.

Next, the NK cell culture method of the present invention comprises: a NK cell stimulation step of treating a culture medium in which peripheral blood mononuclear cells (PBMC) isolated from blood are cultured, with an NK cell stimulation substance; a T cell stimulation step of treating the culture medium with a T cell stimulation substance after the NK cells are stimulated in the NK cell stimulation step; and an additive composition treatment step of treating the culture medium with an additive composition for an NK cell culture medium, the composition containing one or more Treg activity suppressing substances. The method may further include a step of harvesting the NK cells after culturing the NK cells for 11 to 16 days after the additive composition treatment step is performed.

Here, the NK cell stimulation substance is at least one selected from the group consisting of an anti-CD16 antibody, ab anti-CD56 antibody, IL-2, IL-12, IL-18, and the T cell stimulation substance is at least one selected from the group consisting of an anti-CD3 antibody, an anti-CD4 antibody, and an anti-CD28 antibody.

More specifically, when culturing NK immune cells, NK cells are stimulated by NK cell-stimulation substances such as anti-CD16 antibody and anti-CD56 antibody while Th1 cells are stimulated and activated by IL-2. In addition, NK cells and Tc cells are stimulated and activated by Th1 cells stimulated and activated by a T cell stimulation substance such as anti-CD3 antibody. Specifically, NK cells are activated and well cultured when stimulated by IL-12, IL-18, and anti-CD16 antibody, and anti-CD56 antibody. However, when stimulated with anti-CD3 antibody, etc., all of the T cells are stimulated. Therefore, regulatory cells (Tregs) and humoral immune cells such as Th2 cells are also stimulated. These cells do not help activate and proliferate NK cells. Especially, since Treg cells interfere with NK cell activation and proliferation. Therefore, when Treg cells are incapacitated, the proliferation rate of NK cells increases.

Among the drugs for inhibiting regulatory T cell (Treg) activity, several types of steroid drugs inhibit T cells, especially Tregs, so they can be effectively used for culturing natural killer cells if used well at the right time. It is carried out on the second day of culture, and the additive composition treatment step may be carried out on the third day of culture. That is, on day 0 and/or day 1 of NK cell culture, NK cells are stimulated by treatment with a NK cell stimulatory substance, and on day 2 of culture, T cells are treated with a T cell stimulatory material such as anti-CD3 antibody, anti-CD4 antibody, or anti-CD28. After activating Tc and NK cells by stimulation, treatment with the additive composition containing the steroid of the present invention on the third day of culture suppresses the immunosuppressive activity of CD4 + T cells, particularly regulatory T cells (Treg), so NK Cells, etc. are proliferated and/or activated, but do not exhibit inhibitory activity. In addition, the additive composition treatment step may be performed more than once after 5 days of culture.

As described above, through the experimental examples, it is confirmed that when the activity of CD4+ T cells, particularly regulatory T cells (Tregs), are inhibited by the NK cell culture method of the present invention, the ratio of NK cells is significantly increased compared to the case where the treatment using the additive composition is not performed.

On the other hand, the additive composition used in the NK cell culture method of the present invention contains a Treg activity suppressing substance at a concentration of 0.1 ug/mL or more. According to one embodiment, the steroid is dissolved in a cytokine 1-1 solution or carrier. The cytokine 1-1 solution contains IL-12 at a concentration of 0.5-5 ng/mL and IL-18 at a concentration of 2-50 ng/mL, and the cytokine 1-1 solution is prepared by dissolving IL-12 and IL-18 in a basic solution. The basic solution (hereinafter referred to as B solution) includes IL-2, L-glutamine, and a cell culture medium.

Next, the immune cell therapeutic agent of the present invention includes the NK cells obtained by the above-described NK cell culture method as an active ingredient. The immune cell therapeutic agent of the present invention may be administered to the patient's body in a Ringer's form by prepared by introducing the NK cells in a carrier such as physiological saline. The immune cell therapeutic agent may further include known ingredients that do not affect the NK cells but are effective for the treatment of the disease of the patient. Here, the patients include may mammal animals, including humans.

In addition, the NK cell reactivation method of the present invention is a method for increasing the titer of NK cells with reduced vitality. Herein, the NK cells with reduced vitality will be referred to as exhausted NK cells. The exhausted NK cells means NK cells that are frozen and then thawed after being cultured for more than 20 days by the NK cell culture method described above. The NK cell reactivation method includes a step of preparing the exhausted NK cells; and culturing the prepared NK cells in a cytokine 1-1 solution.

In the early stages of lymphocyte cell culture, that is, when the cells are young (initial culture), the proliferation rate thereof is high at high concentrations of cytokines such as IL-2. However, in the later stages, for example, after the fifth, ninth, or tenth day of culture, the proliferation rate drops. That is, the lymphocyte cells do not grow well. In this case, when the concentration of cytokines is lowered, the immunosuppressive activity of Tregs is lowered, and thus the proliferation rate of the lymphocyte cells is increased again. However, in this case, there is a problem in that the proliferation rate of NK cells is not high and weak NK cells tend to lose their activity. At this time, when the culture medium is treated with a solution containing cytokines such as IL-2, IL-12, and IL-18, the activity of NK cells is increased with the disadvantage in which Tregs are also activated. Due to the activation of Tregs, the population of NK cells decreases and it is not easy to activate NK cells consistently. When Tregs are weak, NK cells can be activated to some extent. However, in most cases, the number of NK cells is rapidly reduced, or NK cells are not activated at all, especially in patients with severe cancer stages. However, when the NK cell culture method of the present invention is used, the NK cell proliferation is not interfered by Treg cells, and the NK cells can proliferate and activate well. In the NK cell culture method of the present invention, NK cells and T cells are stimulated at the initial stage of culture. Next, Tregs are then suppressed by the addition of an additive composition containing a steroid. In this state, the concentration of cytokines is increased on or after the eighth, ninth, or tenth day of culture. In this case, the NK cell proliferation and activation can be performed well.

In particular, when the reactivation method of the present invention is used, it was confirmed that even old-age NK cells that have reached the last stage of cell division after culturing of three to four weeks and have almost lost their activity were also well activated.

Next, the NK cell culture medium composition preparation method of the present invention is a method for obtaining a culture medium composition containing a large amount of IFN-γ and IL-10. The method includes the step of: isolating NK cells that are cultured for 11 days by the NK cell culture method; culturing the isolated NK cells in a cytokine 1-1 solution; and harvesting the NK cells and obtaining the remaining culture medium composition. That is, it is because IL-10 is known to play a role in eliminating inflammation by inducing macrophages to change to M2b type.

Next, the cosmetic composition and pharmaceutical composition of the present invention use the culture medium composition obtained through the NK cell culture medium composition preparation method, as an active ingredient. In particular, it was confirmed that the culture medium composition of the present invention contained IFN-γ and IL-10 in large amounts through as the experimental examples to be described later. Therefore, the cosmetic composition and pharmaceutical composition are effective not only in alleviating skin troubles but also in treating wounds and skin diseases. In addition, according to Kores Patent Application Publication No. 10-2018-0057359(KR10-2018-0057359) in which the effects of the cell culture medium composition obtained during NK cell culture is described, the cell culture medium composition is effective in at least one of skin whitening, pigmentation removal, and wrinkle reduction, and also effective in treating inflammatory diseases such as allergic rhinitis, allergic dermatitis, atopic dermatitis, acne, and inflammation caused by insect bites, etc.. Especially, the cell culture medium composition have an effect of quickly relieving itching. In addition, the cell culture medium composition effectively treat burns and wounds, thereby having has good skin recovery and pigmentation removal effects. Therefore, the effectiveness of the cosmetic composition and pharmaceutical composition of the present invention is indirectly supported by the disclosure of the patent literature.

In addition, a medium addition kit for immune cell culture (NKTM) used in some examples of the NK cell culture method of the present invention to be described later is individually packaged so that it can be added to the culture medium at each stage of immune cell culture. The medium addition kit includes a B unit (also called basic solution or B solution), a C1-1 unit (cytokine 1-1 solution), a C1-2 unit, a C2 unit, an A1 unit, an A2 unit, and a D unit with different components. Detailed description of each of the units and detailed description of specific conditions for culturing immune cells (NKTM culturing process) using the units are disclosed in Kores Patent Application Publication No. 10-2018-0057359 (hereinafter referred to as "the prior patent"). Therefore, a description that will be given blow will be focused on only the parts different from those described in the prior patent.

### Example 1

1. Preparation of B solution
   2.2 mg of IL-2 and 500 mM of an L-glutamine solution were dissolved in a basal medium for suspension cell culture to prepare 10L of a B solution. When IL-2 or L-glutamine is already present in the basal medium, the amount of IL-2 or L-glutamine solution to be added to the basal medium was adjusted to meet the final concentration.
2. Preparation of cytokine 1-1 solution
   20 ug of IL-2 and 125 ug of IL-18 were dissolved in distilled water to prepare 10 mL of cytokine solution (C solution). 1 mL of the C solution was dissolved in 1000 mL of the basic solution (B1 solution) to prepare a cytokine 1-1 solution (C1-1 solution).
3. Preparation of additive composition for NK cell culture
   Additive Composition 1 for an NK cell culture medium was prepared by dissolving dexamethasone sodium phosphate in the cytokine 1-1 solution to be a concentration of 1 ug/mL.

### Example 2

Prednisolone (Sorondo Tablet, 5 mg/tablet, Yuhan Corporation) was dissolved in an RPMI medium in an amount corresponding to a concentration of 167 ug/mL, and the resulting solution was then diluted 20-fold with the C1-1 solution to obtain Additive Composition 2 having a concentration of 8.3 ug/mL for an NK cell culture medium.

### Example 3

Methylprednisolone (PD tablet, 4mg/tablet, Choongwae Pharmaceutical) was dissolved in an RPMI medium in an amount corresponding to a concentration of 80 ug/mL, and the resulting solution was diluted 10-fold with the C1-1 solution to prepare Additive Composition 3 with a concentration of 8 ug/mL for an NK cell culture medium.

### Example 4

As betamethasone sodium phosphate (Hanol betamethasone, 5.2mg/1mL ampule, Hanol Biopharma), 1 uL of Hanol betamethasone was used. The 1 uL of Hansol betamethasone was dissolved in 10mL of the C1-1 solution to prepare Additive Composition 4 with a concentration of 0.4 ug/mL for an NK cell culture medium.

### Example 5

NK cells were cultured as follows.
1. Preparation of medium addition kit
   (1) A solution: A solution prepared by dissolving an anti-CD16 antibody and an anti-CD56 antibody in the B1 solution so that each of the anti-CD16 antibody and anti-CD56 antibody is contained at a concentration of 0.01 to 1.5 ug/mL.
   (2) A1 solution: A solution prepared by dissolving an anti-CD16 antibody in the C1-1 solution so that the anti-CD16 antibody is contained at a concentration of 0.1 ~ 15 ug/mL.
   (3) A2 solution: A solution prepared by dissolving an anti-CD56 antibody in the C1-1 solution so that the anti-CD56 antibody is contained at a concentration of 0.1 to 15 ug/mL.
   (4) B1 solution: A solution prepared by dissolving IL-2 in a basic medium for suspension cell culture so that the IL-2 is contained at a concentration of 1000 to 4000 IU/mL.
   (5) B2 solution: A solution in which the concentration of IL-2 is two times higher compared to the B1 solution.
   (6) C1-1 solution: A solution prepared by dissolving IL-12 and IL-18 in the B1 solution so that the IL-12 is contained at a concentration of 0.5 to 5 ng/mL and the IL-18 is contained at a concentration of 2 to 50 ng/mL.
   (7) C5 solution: A solution in which the concentrations of IL-2 and IL-18 are five times higher than the C1-1 solution.
   (8) D solution D: A solution prepared by dissolving an anti-CD3 antibody in the C1-1 solution so that the anti-CD3 antibody is contained at a concentration of 1 to 12 ug/mL.
   (9) R solution: A basic medium solution for suspension cell culture, the solution containing L-glutamine at a concentration of 3 to 12 mM but not containing a cytokine or antibody.
2. Cultivation process
   Lymphocyte extraction and autologous plasma preparation were performed in the same manner as in the prior patent.
   Day 0: 1.0 X 10⁷ PBMCs isolated from blood were dissolved in the C1-1 solution to start culture.
   Day 1: 1 mL of the A2 solution was added to the cells being cultured.
   Day 2: 1 mL of the D solution was added to the cells being cultured.
   Day 3: Additive Composition 1 for an NK cell culture medium was added in an amount of 5 mL to the cells being cultured.
   Day 4: If necessary, the cells were transferred to a larger culture vessel and then incubated there. 3 mL of the R solution was added to the cells being cultured, or the culture medium was replaced with 10 mL of the C1-1 solution.
   Day 5: 20 mL of the A1 solution was added to the cells being culture.
   Day 6: 20 mL of the A solution was added to the cells being cultured.
   Day 7: 40 mL of the B1 solution was added to the cells being cultured.
   Day 8: 300 mL of the R solution was added to the cells being cultured.
   Day 10: 300 mL of the R solution was added to the cells being cultured.
   Day 11: 300 mL of the B2 solution and 50 mL of the C5 solution were added to the cells being cultured.
   Day 14 to Day 19: The cells were harvested.

### Example 6

NK cells were cultured in the same manner as in Example 5, except that 4.5 mL of Additive Composition 2 for an NK cell culture medium was added on Day 3.

### Example 7

NK cells were cultured in the same manner as in Example 5, except that 4.5 mL of Additive Composition 3 for an NK cell culture medium was added on Day3.

### Example 8

NK cells were cultured in the same manner as in Example 5, except that 4.5 mL of Additional Composition 4 for an NK cell culture medium was added on Day3.

### Comparative Example 1

NK cells were cultured in the same manner as in Example 5, except that Additive Composition 1 for an NK cell culture medium was not added on Day 3.

### Experimental Example 1

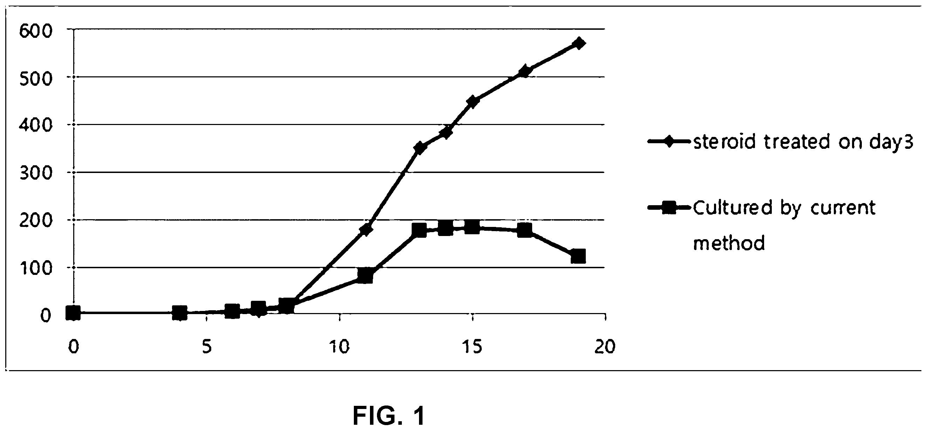

In order to confirm the effect of an additive composition for an NK cell culture medium containing one or more Treg activity suppressing substances as an active ingredient upon NK cell culture, the result of cell proliferation for the case where NK cells were cultured in the same manner as in Example 5 and the result of cell proliferation for the case where NK cells were cultured in the manner as in Comparative Example 1 were observed. The results of the observation are shown in Table 1 (unit: ×10⁷ pieces), Table 2, and FIGS. 1 to 2B.

**[Table 1]**

| Day | 0 | 4 | 6 | 7 | 8 | 11 | 13 | 14 | 15 | 17 | 19 |
|---|---|---|---|---|---|---|---|---|---|---|---|
| Example 5 | 1.0 | 0.89 | 4.6 | 7.0 | 13.5 | 178 | 352 | 384 | 447 | 512 | 570 |
| Compara tive Example 1 | 2.0 | 1.45 | 6.4 | 10. 0 | 19.1 | 78 | 175 | 180 | 182 | 175 | 120 |

**[Table 2]**

| Culturing Method | NK cell (%) | NK cell activity (%, effector cell/K562) | |
|---|---|---|---|
| | | 30:1 | 10:1 |
| Example 5 | 97.08 | 91.4 | 79.6 |
| Comparative Example 1 | 92.55 | 83.5 | 72.3 |

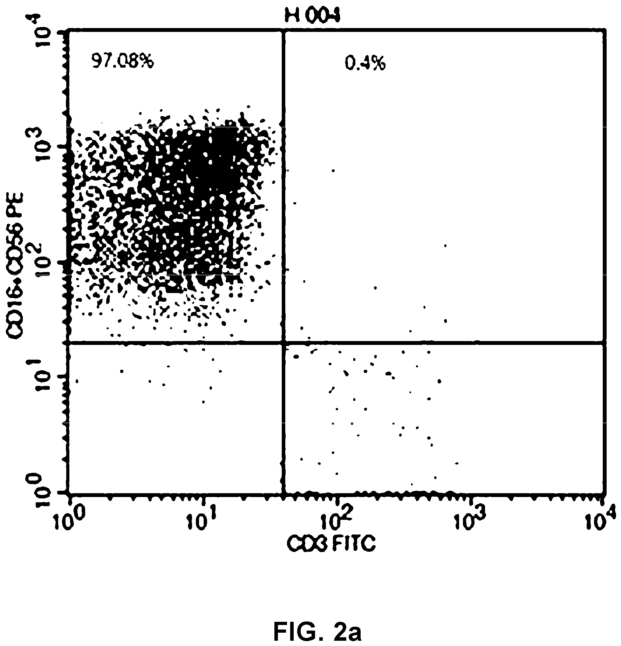

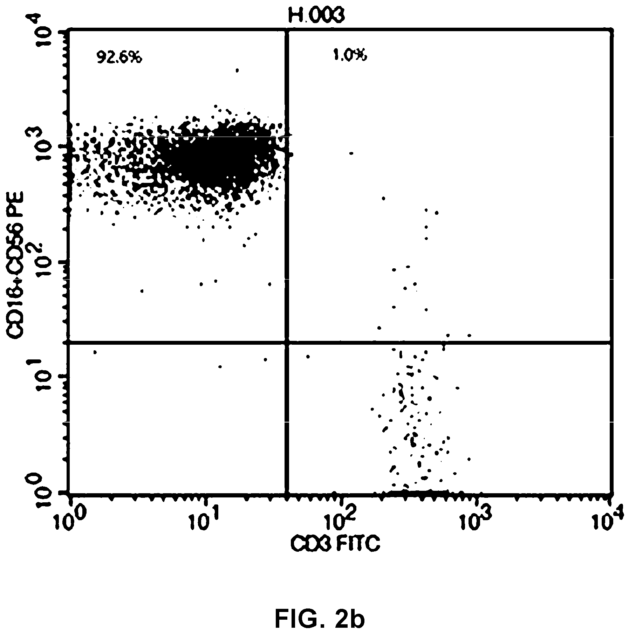

As can be seen from Table 1 and FIG. 1, in the process of culturing immune cells, Th cells were stimulated by treatment with an anti-CD3 antibody on Day 2. On Day 3, an additive composition for an NK cell culture medium, containing 1 uL of dexamethasone (a steroid injection), was added to the culture medium as in Example 5. On day 4, the existing culture medium was replaced with the C1 solution to eliminate cytokines generated by Th2 cells and Treg cells and then the NK cells were cultured in the C1 solution thereafter. Then, the number of NK cells obtained through the method of Example 5 and the number of NK cells obtained through the method of Comparative Example 1 in which a steroid was not used were compared. The comparison revealed that the proliferation rate of the NK cells cultured by the method of Example 5 was 4.3 or more times higher than by the method of Comparative Example 1 when cultured for 14 days, and 5.9 or more times higher when cultured for 17 days. In addition, as can be seen from Table 2 and FIGS. 2A and 2B, when NK cells were cultured by the method of Example 5, the ratio of NK cells to all the cells in the culture medium and the activation degree of NK cells were higher than the case where NK cells were cultured by the method of Comparative Example 1. When using the method of Example 5, the effect may be better if a steroid is added once more after the medium replacement on Day 4. However, it can be seen that the effect is sufficiently good even in the case where the steroid treatment was performed only once on Day3.

In addition, in the conditions in which activated lymphocytes obtained by lymphocyte culture were used as effector cells and a blood cancer cell line (K562) was used as target cells, and the ratio of activated lymphocytes to cancer cells was set to 10:1, the titer analysis was performed to measure the killing ability of activated lymphocytes against a blood cancer cell line. Since it is natural that the higher the ratio of NK cells to the activated lymphocytes, the higher the value of the killing ability, it can be predicted that the therapeutic effect of activated lymphocytes in which the ratio of NK cells cultured by the culturing method of the present invention is excellent.

### Experimental Example 2

To confirm the effect of the additive composition for an NK cell culture medium, containing various steroids as a Treg activity suppressing substance, the cell proliferation results obtained by the NK cell culture performed according to the methods of Examples 6 to 8 were observed. The observation results are shown Table 3 (unit: ×10⁷ pieces).

**[Table 3]**

| Day | 0 | 4 | 6 | 7 | 8 | 11 | 13 | 14 | 15 | 17 | 19 |
|---|---|---|---|---|---|---|---|---|---|---|---|
| Example 6 | 1.0 | 0.7 | 3.1 | 6.6 | 13.5 | 168 | 312 | 365 | 395 | 462 | 505 |
| Example 7 | 1.0 | 0.6 | 3.2 | 6.5 | 13.2 | 153 | 325 | 374 | 420 | 480 | 520 |
| Example 8 | 1.0 | 0.7 | 3.2 | 6.9 | 14.2 | 172 | 330 | 385 | 452 | 515 | 530 |

Instead of dexamethasone contained in Additive Composition 1 for an NK cell culture medium, Additive Composition 2 for an NK cell culture medium contains prednisolone, Additive Composition 3 contains methylprednisolone, and Additive Composition 4 contains betamethasone. As can be seen from Table 3, in Examples 6, 7, 8 in which Additive Composition 2, Additive Composition 3, and Additive Composition 4 were used, respectively, instead of Additive Composition 1, the NK cell proliferation were very good.

### Experimental Example 3

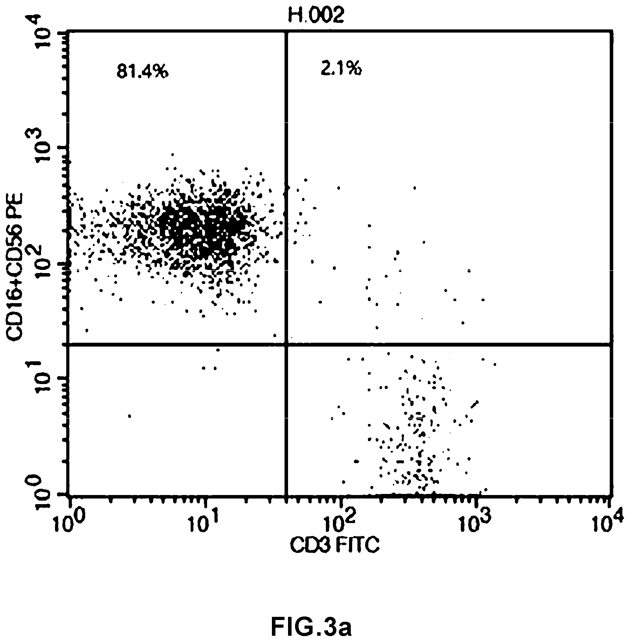

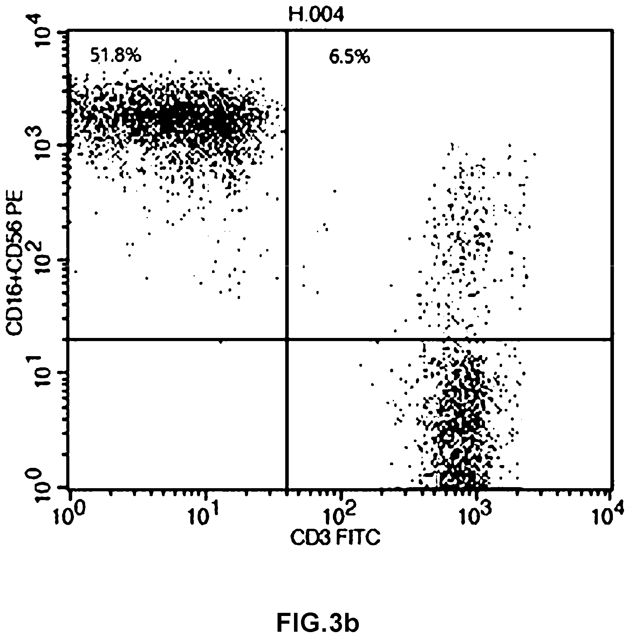

Cells were cultured in the same manner as in Example 5 except that cells having a NK cell ratio of about 50% were used, the concentration of the steroid contained in the additive composition for an NK cell culture medium was increased by about 2 times, and the additive composition was added on Day 3, Day 4, and Day 8 during the cell culture. In addition, cells were cultured in the same manner as in Comparative Example 1 except that cells having an NK cell ratio of about 50% was used. The proliferation results of the cells for the respective cases are shown in FIGS. 3A and 3B.

FIGS. 3A and 3B show that when the additive composition of the present invention is used, the NK cell ratio is 81.35% whereas when not used, the NK cell ratio is 51.76%. Even when NK cell culture starts in a condition in which the NK cell ratio is high, the NK cell proliferation ratio can be increased when the additive composition of the present invention is added during the NK cell culture.

### Experimental Example 4

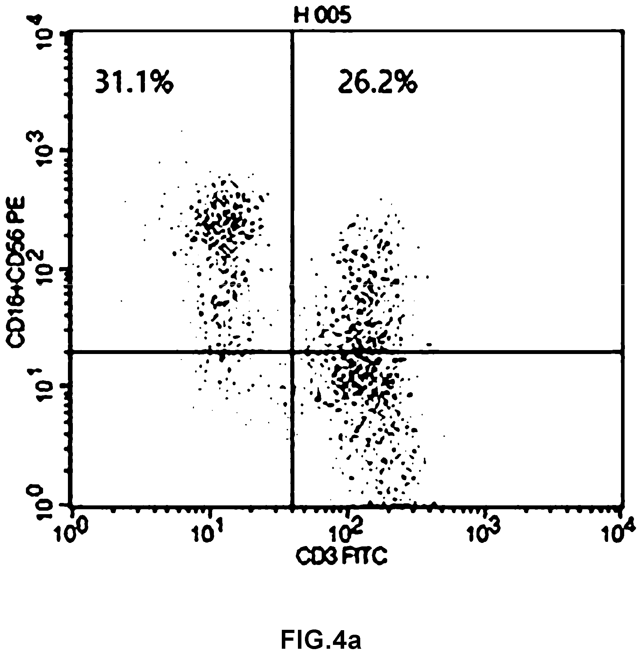

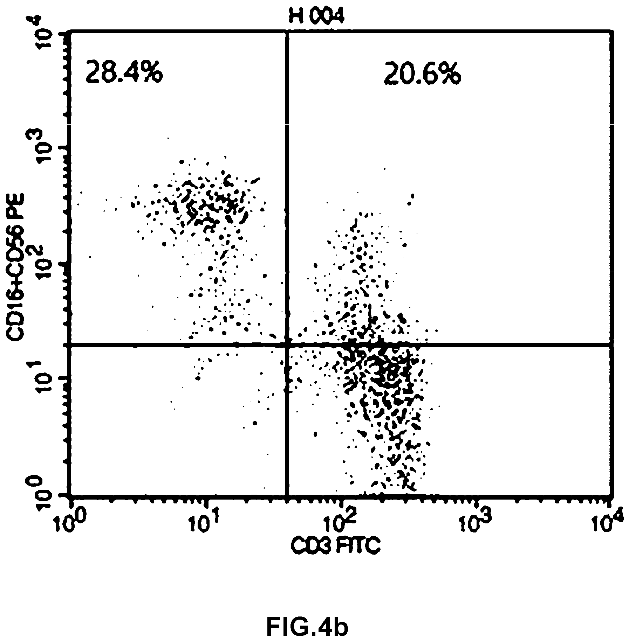

To confirm the effect of the additive composition for an NK cell culture medium, of the present invention, NK cells were cultured by a typical NK cell culture method, without using an NK cell culture addition kit as in Examples. Lymphocytes (1 X 10⁷) were isolated. Next, the cells were stimulated using cytokines such as IL-2, IL-12, and IL-18 that are used to stimulate NK cells, and then stimulated with an anti-CD3 antibody on the second day of culture. In this example, CD16 antibody and CD56 antibody were not used at all. On the third day of culture, the culture medium was treated with Additive Composition 1 for an NK cell culture medium. Afterwards, a normal medium supplemented with IL-2 was used as the culture medium, and the medium was added as the number of cells increased during the cell culture. The cell culture results obtained after culturing the cells for 14 days are shown in Table 4, FIGS. 4A and 4B.

Table 4 and FIGS. 4A and 4B show that when the additive composition for an NK cell culture medium, of the present invention, is added to the culture medium on the third day of culture during NK cell culture, even in the case where NK cells are cultured with the use of only cytokines and anti-CD3 antibodies without using an additional medium addition kit, the NK cells proliferated more than twice than the case where the additive composition of the present invention is not used. In addition, the proportion of NK cells is 31.1% that is higher than that of the case where the additive composition is not used, and the proportion of NKT cells is also as high as 26.2%.

**[Table4]**

| Method | Number of cells(X 10⁷) | | FACS data after culture | | |
|---|---|---|---|---|---|
| | Initial | After 14-day culture | NK cell(%) | NKT cell (%) | NK+NKT(%) |
| Typical method | 1.0 | 215 | 28.4 | 20.6 | 49.0 |
| Use of additive composition for NK cell culture medium | 1.0 | 510 | 31.1 | 26.2 | 57.3 |

### Experimental Example 5

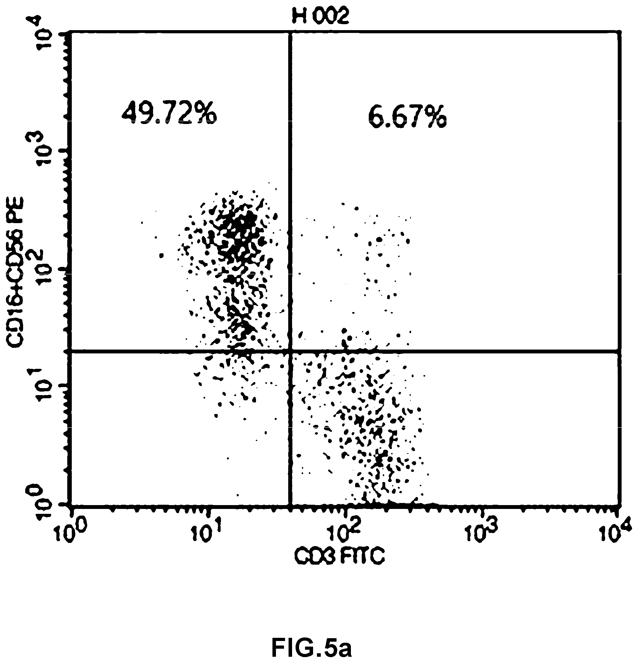

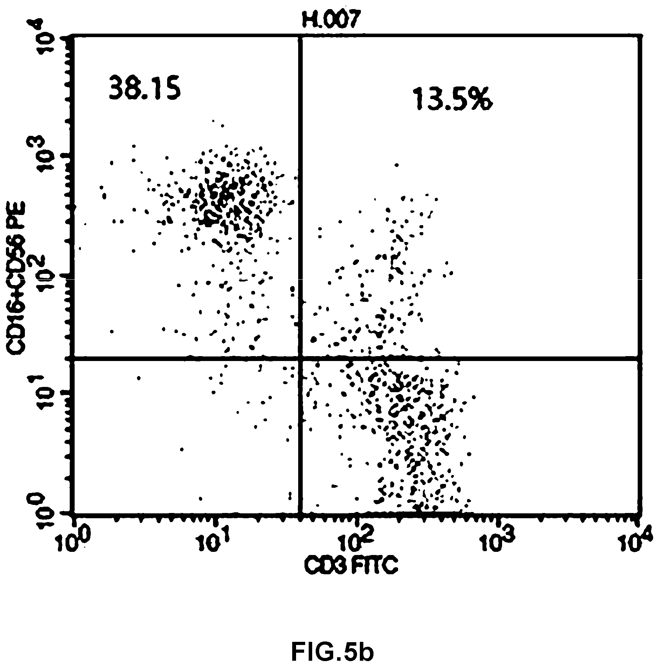

To confirm the effect of the additive composition for an NK cell culture medium, of the present invention, cells were cultured using a method described below, without using the NK cell culture addition kit as in Examples. That is, NK cells were cultured using a conventional NK cell culture method in which an immobilized anti-CD3 antibody used. The anti-CD3 antibody was immobilized in a T25 flask, and isolated lymphocytes (0.8 X 10⁷) were reacted for about 1 hour and then cultured using IL-2, CD16, and CD56 antibodies. The CD16 and CD56 antibodies were stimulated on the second day, the fourth day, and the fifty day, and on the third day of culture, Additive Composition 1 for an NK cell culture medium was added. After that, a normal culture medium supplemented with IL-2 was used. The cells were cultured while adding the medium as the number of cells increased. After the cells were cultured for 14 days, the results are observed. The results are shown in Table 5, FIGS. 5A and 5B.

Table 5 and FIGS. 5A and 5B show the result of NK cell culture in the case where an anti-CD3 antibody was immobilized in a T25 flask without using an additional medium addition kit, and NK cells were cultured using a cytokine, an anti-CD16 antibody, and an anti-56 antibody. Even in this case, when the additive composition of the present invention for an NK cell culture medium was added to the culture medium on the third day of culture, the NK cells proliferated more than twice after 14 days of culture compared to the case where the additive composition of the present invention was not used. In addition, the specific gravity of the NK cell considerably increased to 49.72%.

**[Table 5]**

| Method | number of cells (X 10⁷) | | FACS data after culture | | |
|---|---|---|---|---|---|
| | Initial | After culture for 14 days | NK cell (%) | NKT cell (%) | NK+NKT (%) |
| Anti-CD3 antibody immobilization | 0.8 | 195 | 38.15 | 13.5 | 51.65 |
| Use of additive composition for NK cell culture medium | 0.8 | 480 | 49.72 | 6.67 | 56.39 |

### Example 9

To evaluate the effect of the additive composition for an NK cell culture medium, of the present invention, on the reactivation of cells with reduced vitality (i.e., exhausted cells), old-aged cells that are cultured for 25 are re-cultured while suppressing Treg cells by using the method of Example 5. Reactivation was attempted as follows. To remove the existing culture medium, the supernatant was removed by centrifugation, the remaining deposit was put in a normal medium (RPMI) and cultured for about 2 hours, and the medium was replaced with the C1 solution and cultured for 3 days.

### Experimental Example 6

The activity of NK cells reactivated in Example 9 was measured, and the results are shown in Table 6.

As shown in Table 6 below, it was observed that the activity was not inhibited by Treg but increased, and the number of cells was not rapidly decreased. However, although it can be considered as a measurement error, a slight decrease in the number of cells was observed to the extent that the number of cells naturally decrease due to aging. This method can activate NK cells within a very short period of time (for example, 1 to 3 days) . This method can activates not only old cells but also NK cells in PBMCs directly isolated from peripheral blood. The method is expected to be very effective in activating NK cells that have lost vitality due to freezing and thawing.

**[Table 6]**

| | Old immune cells cultured for 25 days | | After 3-day culture with C1 solution treatment | |
|---|---|---|---|---|
| Cell | NK cells(%) | Titer (10:1, K562) | NK cells(%) | Titer (10:1, K562) |
| 1 | 57.74 | 7.3 | 69.57 | 74.51 |
| 2 | 55.52 | 41.67 | 58.06 | 92.22 |
| 3 | 55.89 | 54.13 | 57.64 | 91.75 |
| 4 | 61.41 | 66.03 | 63.44 | 92.86 |

### Example 10

NK cells were cultured in the same manner as in Example 5 except that PBMCs were obtained from a person with a high concentration of IL-10 in the blood that Treg cells are likely to be highly activated. On Day 12, the cells were taken out, the existing culture medium was removed, and the cells were cultured with the C1 solution for 2 days. Next, the NK cells were isolation, and the remaining culture medium composition was obtained.

### Experimental Example 7

The components of the culture medium composition obtained in Example 10 were identified, and the results are shown in Table 7.

**[Table 7]**

| Cytokines | IFN-γ (ng/mL) | IL-8 (ng/mL) | IL-10 (pg/mL) | Notes |
|---|---|---|---|---|
| A | 150.24 | 0.29 | 912.21 | Activation with C1 solution |
| B | 3.75 | 0.32 | 290.57 | No activation with C1 solution |

Table 7 shows that stimulation with the C1 solution on the 12th day of culture and subsequent two-day culture considerably increased the number of cells and significantly increased IFN-γ and IL-10. In this case, the amount of generated IL-8 was small. On the other hand, when the cells were isolated without activation with the C1 solution, the overall increased in the cytokine content was small. As can be seen from the experimental example, when even human immune cells in which Treg cells are present in a large number are treated with the additive composition for an NK cell culture medium, of the present invention, and then stimulated with the C1 solution on the 12^{th} day of culture, the number of the human immune cells are not reduced but rather increased, and a large amount of IFN-γ and IL-10 is generated. On the other hand, it is known that IL-8 is a cytokine that attracts neutrophils and is highly related to inflammation, and IL-10 is known to play a role in removing inflammation by inducing macrophages to change to M2b type. Therefore, it is considered that the culture medium composition thus obtained is effective in treating skin diseases caused by inflammation.

Although the present invention has been illustrated and described with reference to preferred embodiments as described above, the present invention is not limited to the above-described embodiments, and those who are ordinarily skilled in the art will appreciate that various changes and modifications thereto are possible without departing form the spirit of the present invention.

## Claims

1. An additive composition for an NK cell culture medium, the additive composition comprising one or more Treg activity suppressing substance as an active ingredient.

2. The additive composition of claim 1, wherein the active ingredient is a steroid and functions to increase the proliferation rate of NK cells.

3. The additive composition of claim 1, wherein the steroid is glucocorticoids.

4. The additive composition of claim 2, wherein the glucocorticoid is one or more substances selected from the group consisting of cortisol, cortisone, prednisolone, prednisone, methylprednisolone, dexamethasone, betamethasone, triamcinolone, fludrocortisone acetate, and deoxycorticosterone acetate.

5. The additive composition of claim 1, wherein treatment with the additive composition is performed after T cells in the NK cell culture medium are stimulated during NK cell culture.

6. The additive composition of any one of claims 1 to 5, wherein the steroid is contained at a concentration of 0.2 µm/ml or higher.

7. An NK cell culture method comprising:
an NK cell stimulation step of treating a culture medium in which peripheral blood mononuclear cells (PBMCs) isolated from blood are cultured, with an NK cell stimulation substance;
a T cell stimulation step of treating the culture medium with a T cell stimulation substance after NK cells are stimulated in the NK cell stimulation step;
an additive position treatment step of treating the culture medium with an additive composition for an NK cell culture medium after the T cells are stimulated in the T cell stimulation step, the additive composition comprising one or more Treg activity suppressing substances.

8. The method of claim 7, wherein the NK cell stimulation substance is at least one selected from the group consisting of an anti-CD16 antibody, an anti-CD56 antibody, and IL-2, IL-12, IL-18, and
the T cell stimulation substance is at least one selected from the group consisting of an anti-CD3 antibody, an anti-CD4 antibody, and an anti-CD28 antibody.

9. The method of claim 7, wherein the Treg activity suppressing substance is a steroid, and
the steroid inhibits the immunosuppressive activity of regulatory T cells (Tregs), thereby helping the proliferation of the NK cells.

10. The method of claim 7, wherein the T cell stimulation step is performed on the second day of culture, and
the additive composition treatment step is performed on the third day of culture.

11. The method of claim 10, wherein the additive composition treatment step is performed again one or more times on or after the fifth day of culture.

12. The method of claim 7, wherein the additive composition comprises the steroid at a concentration of 0.2 ug/mL or more, and
the steroid is dissolved in a carrier or cytokine 1-1 solution, wherein the cytokine 1-1 solution is obtained by dissolving IL-12 and IL-18 in a basic solution such that the IL-12 and the IL-18 are contained at a concentration of 0.5-5 ng/mL and a concentration of 2 of 50 ng/mL, respectively in the basic solution.

13. The method of any one of claims 7 to 12, further comprising:
a step of harvesting the NK cells after culturing the NK cells for 11 to 16 days after performing the additive composition treatment step.

14. An immune cell therapeutic agent comprising NK cells obtained by the NK cell culture method of claim 13 as an active ingredient.

15. An NK cell reactivation method comprising:
preparing exhausted NK cells resulting from NK cells that are separated after being cultured for 20 days or more by the NK cell culture method of any one of claims 7 to 12, or preparing exhausted NK cells resulting from NK cells that are separated after being cultured for 20 days or more by the NK cell culture method of any one of claims 7 to 12 , or NK cells that are harvested, frozen and then thawed;; and
culturing the prepared exhausted NK cells in a cytokine 1-1 solution.

16. A method of preparing an NK cell culture medium composition, the method comprising:
separating NK cells that are cultured for 11 days by the NK cell culture method of any one of claims 7 to 12;
culturing the separated NK cells in a cytokine 1-1 solution; and
obtaining a culture medium composition remaining after harvesting the cultured NK cells.

17. A cosmetic composition for alleviating skin troubles, the cosmetic composition comprising the NK cell culture medium composition obtained by the method of claim 16 as an active ingredient.

18. A pharmaceutical composition for the treatment of skin diseases, the pharmaceutical composition comprising, as an active ingredient, the NK cell culture medium composition prepared by the method of claim 16.
